# EUROPEAN PATENT SPECIFICATION

(11) **EP 2 548 881 B1**
(45) Date of publication and mention of the grant of the patent: **17.12.2014**
(21) Application number: 10847665.6
(22) Date of filing: 16.03.2010
(51) Int. Cl.: C07J 53/00

(54) **PREPARATION METHOD OF DROSPIRENONE**
VERFAHREN ZUR HERSTELLUNG VON DROSPIRENON
PROCÉDÉ DE PRÉPARATION DE DROSPIRÉNONE

(43) Date of publication of application: 23.01.2013
(73) Proprietor: Taizhou Taifa Pharmaceuticals Co., Ltd, Zhejiang 317300 (CN)
(72) Inventor: WANG, Jiazhen, Taizhou Zhejiang 317300 (CN)
(74) Representative: Gulde & Partner
(86) International application number: PCT/CN2010/071074
(87) International publication number: WO 2011/113196

(56) References cited:
- EP-A2- 1 903 051
- WO-A2-2008/137050
- WO-A2-2009/059765
- CN-A- 1 223 662
- CN-A- 101 092 443
- CN-A- 101 503 455
- US-A1- 2007 021 603

## Description

### Field of The Invention

The present invention relates to the field of chemical synthesis, especially the preparation method of drospirenone.

### Background of The Invention

Drospirenone has the chemical name 6β,7β,15β,16β-dimethylene-3-oxo-17α-pregn-4-ene-21,17-carbolactone, CAs#:67392-87-4, molecular formula: C₂₄H₃₀O₃, molecular weight: 366.49, and its structural formula is as follows:

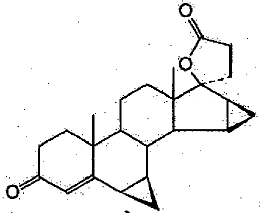

Drospirenone is the fourth-generation progestogen developed by the German Company Schering, initially available in Germany in November, 2000 and now on the markets of United States of America and most European countries. This product has activities as both anti-mineralocorticoid and antiandrogen: the antiandrogen decreases the sebaceous gland activity and the adverse reaction; and the anti-mineralocorticoid lowers excretion of sodium and water, fluid retention and weight gain as well as symptoms associated with menstruation. Meanwhile, drospirenone has little impact on blood pressure or blood lipid, and has the function of losing weight and maintaining health. Many women choose this product for better skin, hair, mood, and more stable weight. As it has these unique characteristics, now drospirenone has become the preferred contraceptive in foreign countries.

European patent EP0075189 discloses a synthetic method of drospirenone as follows:

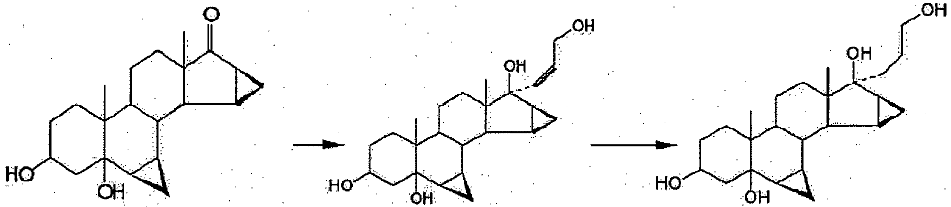

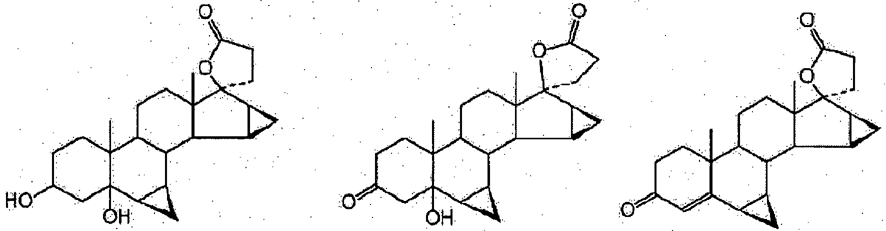

The synthetic method disclosed in the patent has potential safety risk as the explosive combustible catalyst and hydrogen are used. In addition, because the dehydration is conducted under the alkaline condition, this may cause the spiro ring to open and be re-arranged.

Chinese patent CN101092443 discloses a new synthetic method of producing drospirenone as follows:

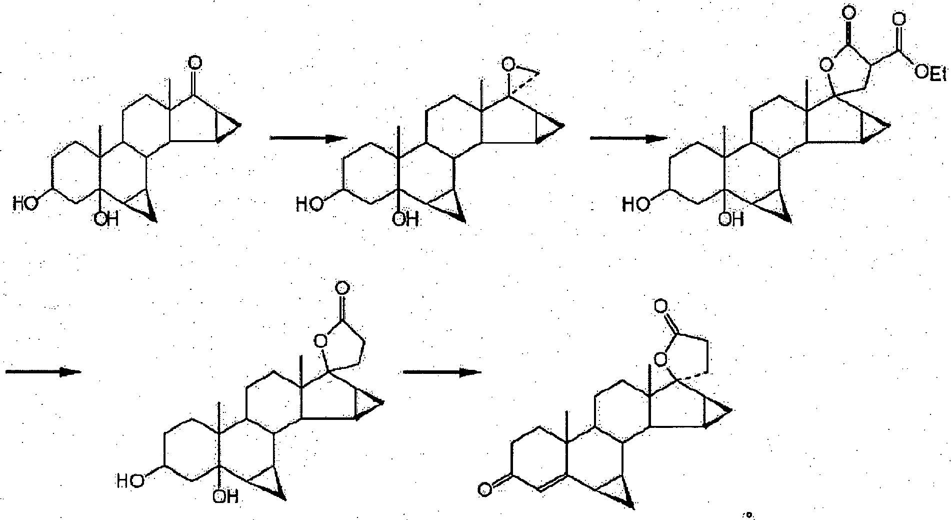

In this method, the dehydration is also conducted under alkaline conditions, this may cause the spiro ring to open and be re-arranged. Moreover, the method is more complicated.

### Summary of The Invention

The present invention provides a preparation method of drospirenone as defined in the claims which has a good reaction specificity to avoid the disadvantages of potential safety risk and easyre-arrangement of the existing methods.

A preparation method of drospirenone comprising the following steps:
3β,5-dihydroxy-6β,7β,15β,16β-dimethylene-5β-androstane-17,20-epoxy of Formula I is taken as starting material (please refer to Chinese patent CN101092443 for the synthetic method of the raw material). The hydroxyl group at the 3-position is oxidized to obtain the oxide of Formula II, and then the ketone group is protected by ketalization to obtain the ketal product of Formula III; the obtained ketal product is converted into the compound of Formula IV by a condensation reaction, and then the lactone compound of Formula V is obtained by decarboxylation; the hydroxyl at the 5-position is subject to sulfonation to obtain the sulfonyl compound of Formula VI; and the deketalization and desulphonation are performed in the reaction system of glacial acetic acid and sodium acetate to produce the 3-keto-4-ene compound, being drospirenone of Formula VII. 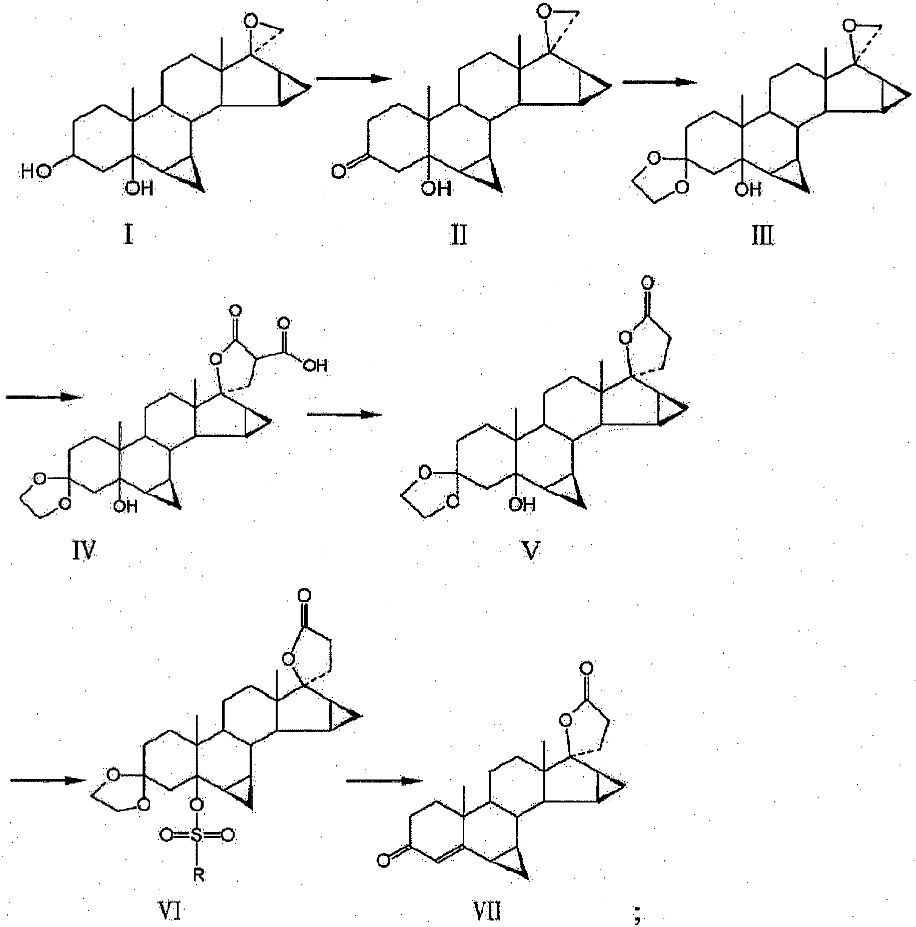
In Formula VI, R is C₁-C₁₀ alkyl, selected from one of phenyl, tolyl and methyl.

The preparation method of drospirenone of the present invention comprises the following steps:
(1) 3β,5-dihydroxy-6β,7β,15β,16β-dimethylene-5β-androstane-17,20-epoxy of Formula I is taken as starting material, and reacts with an oxidant in solvent at 0°C-80°C to obtain the oxide of Formula II;
(2) The oxide of Formula II, glycol and acid catalyst are mixed and subject to reflux reaction or reaction with dehydration agent at 0 °C -80 °C to obtain the ketal product in Formula III, so the product has better hydrophobicity and higher output;
(3) Alcoholic solution of dimethyl malonate is blended with miscible liquids of alcohol and sodium alcoholate at -10 °C to 30 °C (alcoholic solution of dimethyl malonate is preferably dripped into the mixture of alcohol and sodium alcoholate), and subject to heating and reflux reaction. The reactant is cooled down, put into the ketal product of Formula III, and subject to reaction at 0°C -80 °C to produce the compound of Formula IV;
(4) The compound of Formula IV reacts with alkali metal halide salt in the presence of N,N-dimethylformamide and is subject to reflux reaction or reaction at 100°C -180°C to obtain the lactone compound of Formula V;
(5) The lactone compound of Formula V reacts with sulfonyl chloride compound in the presence of pyridine at 0 °C -80 °C to obtain the sulfonyl compound of Formula VI;
(6) The sulfonyl compound of Formula VI is subject to reflux reaction in the reaction system of glacial acetic acid and sodium acetate to produce drospirenone of Formula VII.

The process route can reduce the loss of products during water washing and prevent the adverse reaction, such as spiro ring and propyl ring damage during dehydration due to use of strong acid or alkali dehydration, by adopting sulfonylation - desulphonation method.

Preferably, the oxidant in step (1) is selected from one of sulfuric acid solution containing chromic anhydride, pyridine solution containing chromic anhydride, N-bromosuccinimide and so on.

The sulfuric acid solution containing chromic anhydride or pyridine solution containing chromic anhydride is prepared by mixing chromic anhydride and sulfuric acid or pyridine, and the oxidant solution of routine concentration in the field is applied to the preparation of the said solution.

The solvent is preferably selected from one or more of acetone, pyridine, methanol, and tert-butyl alcohol.

The acid catalyst said in step (2) is preferably selected from one of p-toluenesulfonic acid, boron trifluoride-diethyl ether solution, sulfuric acid, and sodium bisulfate.

The dehydration agent is preferably triethyl orthoformate.

The weight ratio of oxide of Formula II to acid catalyst is preferably 1:0.00005-0.1.

The weight ratio of oxide of Formula II to glycol is preferably 1:2-10.

The reaction in step (2) can also be performed in organic solvents which can be some common solvents such as benzene, methylene chloride and so on.

The alcohol in step (3) is preferably selected from one of methanol, and ethanol; and the sodium alcoholate is preferably selected from one of sodium methoxide, and sodium ethoxide.

The alkali metal halide salt in step (4) is preferably selected from one of lithium bromide, and sodium chloride.

The reaction system in step (4) can also be added with appropriate amount of water

The sulfonyl chloride compound in step (5) is preferably selected from one of benzene sulfonyl chloride, p-toluenesulfonyl chloride, and methane sulfonyl chloride.

The sodium acetate preferably accounts for 9%-20% (mass percent) in the reaction system in step (6).

The reaction time is not strictly limited in the steps (1)-(6) of this invention. It can be considered as an end of the reaction when the reaction of one or a plurality of raw materials are completed by sampling at predetermined time with following up analysis through thin layer chromatography (TLC).

To ensure complete reaction, experiments show the reaction time in step (1) is often 1-3 hours;
The reaction time in step (2) is often 1-20 hours;
The reaction time after the ketal product of formula III is added in step (3) is often 3-25 hours;
The reaction time in step (4) is often 0.5-4 hours;
The reaction time in step (5) is often 0.5-3 hours;
The reaction time in step (6) is often 0.5-1 hours.

The amounts of raw materials in all steps are not strictly limited, generally in accordance with the stoichiometric ratio ofreaction equation (i.e. mole ratio 1:1) or partial excess of raw materials.

The product in this invention has the following advantages:

The process route of the present invention has high intensification, reaction specificity, less by-products and high yield of products in each step, thus overcoming the disadvantages of low yield and unstable quality. Moreover, the production process is very safe and suitable for industrial production.

### Description of The Preferred Embodiments

The preparation method of the present invention is specifically described as follows, which does not limit the invention.

### Example 1

(1) 5g of 3β,5-dihydroxy-6β,7β,15β,16β-dimethylene-5β-androstane-17,20-epoxy (compound of Formula I) was dissolved in 50ml pyridine, added into 50ml pyridine solution containing 5g chromic anhydride, evenly stirred, subject to reaction at 80°C for 1 hour, placed overnight at room temperature, put into ice water, extracted and filtered to obtain 4.5g oxide of Formula II.
(2) 4.5g of the oxide of Formula II, 50ml of dichloromethane, 5ml of triethyl orthoformate were mixed with 10ml glycol, added with 0.0025g -toluenesulfonic acid and stirred at room temperature for 3 hours to obtain 5g ketal product of Formula III.
(3) The solution of 500ml methanol and 20g sodium methoxide is dripped with 70ml methanol solution containing 60ml dimethyl malonate at 0°C, subject to heating reflux reaction for 30 minutes, cooled down to room temperature, added with 20g ketal product of Formula III (20g ketal product of Formula III prepared by step (2)), stirred at room temperature for 3 hours, and then subject to heating and reflux reaction for 10 hours to obtain the compound of Formula IV.
(4) All of compound of Formula IV produced in last step was mixed with 120ml DMF, 20ml H₂O and 24g lithium bromide, and subject to reflux reaction for 3.5 hours to obtain 22g lactone compound of Formula V.
(5) 22g of the lactone compound of Formula V was dissolved in 100 ml pyridine, added with 16g benzene sulfonyl chloride, and subject to reaction at room temperature for 2 hours to with 16g benzene sulfonyl chloride, and subject to reaction at room temperature for 2 hours to obtain the sulfonyl compound of Formula VI.
(6) All of sulfonyl compound of Formula VI produced in the last step was mixed with 110ml glacial acetic acid and 11g sodium acetate, and subject to heating and reflux reaction for 30 minutes to obtain 18g drospirenone of Formula VII.

### Example 2

(1) 5g of 3β,5-dihydroxy-6β,7β,15β,16β-dimethylene-5β-androstane-17,20-epoxy (compound of Formula I) was dissolved in 150ml acetone, added into the solution containing 5g chromic anhydride, 15ml water and 5ml sulfuric acid, evenly stirred, and subject to reaction at 0°C for 3 hours to obtain 4.7g oxide of Formula II.
(2) 5g of the oxide of Formula II (5g oxide in Formula II prepared by step (1)), 150ml of benzene, 10ml of glycol were evenly mixed, added with 0.025g p-toluenesulfonic acid and subject to reflux reaction for 20 hours to obtain 5.1g ketal product of Formula III.
(3) The solution of 500ml ethanol and 20g sodium ethoxide was dripped with a 70ml ethanol solution containing 60ml dimethyl malonate at 0 °C, subject to heating and reflux reaction for 20 minutes, cooled down to room temperature, added with 20 g ketal product in Formula III (20g ketal product in Formula III prepared by step (2)), stirred at room temperature for 3 hours, and then subject to heating and reflux reaction for 20 hours to obtain the compound of Formula IV.
(4) All of compound of Formula IV produced in last step was mixed with 120ml DMF, 20ml H₂O and 24g sodium chloride, and subject to reflux reaction for 3.5 hours to obtain 21.5g lactone compound of Formula V.
(5) 20g of the lactone compound of Formula V was dissolved in 100 ml pyridine, added with 16g p-toluenesulfonic acid chloride, and subjected to reaction at room temperature for 2 hours to obtain the sulfonyl compound of Formula VI.
(6) All of sulfonyl compound of Formula VI produced in the last step was mixed with 150ml glacial acetic acid and 15g sodium acetate, and subject to heating and reflux reaction for 60 minutes to obtain 17.2g drospirenone of Formula VII.

### Example 3

(1) 10g of 3β,5-dihydroxy-6β,7β,15β,16β-dimethylene-5β-androstane-17,20-epoxy (compound of Formula I) was dissolved in 150ml tertiary butanol and 150ml methanol, added with 2g N-bromsucciniamide and appropriate amount of water, evenly stirred, and subject to reaction at room temperature for 3 hours to obtain 8.5g oxide of Formula II.
(2) 8.5g of the oxide of Formula II, 5ml of triethyl orthoformate, and 50ml of glycol were evenly mixed, added with 0.0005g boron trifluoride-ether, and stirred at room temperature for 3 hours to obtain 9.5g ketal product of Formula III.
(3) The solution of 500ml ethanol and 20g sodium ethoxide was dripped with 70ml ethanol solution containing 60ml dimethyl malonate at 0°C, subject to heating and reflux reaction for 20 minutes, cooled down to room temperature, added with 8g ketal product of Formula III, stirred at room temperature for 3 hours, and then subject to heating and reflux reaction for 20 hours to obtain the compound of Formula IV.
(4) All of compound of Formula IV produced in last step was mixed with 120ml DMF, 20ml H₂O and 24g sodium chloride, and subject to reflux reaction for 3.5 hours to obtain 8.5g lactone compound of Formula V.
(5) 20g of the lactone compound of Formula V (20g lactone compound of Formula V prepared by step (4)) was dissolved in 100 ml pyridine, added with 10g methane sulfonyl chloride, and subject to reaction at 0°C for 2 hours to obtain the sulfonyl compound of Formula VI.
(6) All of sulfonyl compound of Formula VI produced in the last step was mixed with 150ml glacial acetic acid and 30g sodium acetate, and subject to heating and reflux reaction for 45 minutes to obtain 16g drospirenone of Formula VII.

### Example 4

(1) 5g of 3β,5-dihydroxy-6β,7β,15β,16β-dimethylene-5β-androstane-17,20-epoxy (compound of Formula I) was dissolved in 50ml pyridine, added into 50ml pyridine solution containing 5g chromic anhydride, evenly stirred, subject to reaction at 80°C for 1 hour, placed overnight at room temperature, put into ice water, extracted and filtered to obtain 4.4g oxide of Formula II.
(2) 5g of the oxide of Formula II (5g oxide of Formula II prepared by step (1)), 50ml of dichloromethane, 5ml of triethyl orthoformate were evenly mixed with 10ml glycol, added with 0.025g sulfuric acid and stirred at room temperature for 3 hours to obtain 4.4g ketal product of Formula III.
(3) The solution of 500ml ethanol and 20g sodium ethoxide was dripped with 70ml methanol solution containing 60ml dimethyl malonate at 0 °C, subject to heating and reflux reaction for 30 minutes, cooled down to room temperature, added with 4 g ketal product of Formula III, stirred at room temperature for 3 hours, and then subject to heating and reflux reaction for 20 hours to obtain the compound of Formula IV.
(4) All of compound of Formula IV produced in last step was mixed with 30ml DMF, 30ml H₂O and 6g lithium bromide, and subject to reflux reaction for 3.5 hours to obtain 4.2g lactone compound of Formula V.
(5) 10g of the lactone compound of Formula V (10g lactone compound of Formula V prepared by step (4)) was dissolved in 50 ml pyridine, added with 6g methane sulfonyl chloride, and subject to reaction at 5°C -10 °C for 0.5h to obtain the sulfonyl compound of Formula VI.
(6) All of sulfonyl compound of Formula VI produced in the last step was mixed with 150ml glacial acetic acid and 20g sodium acetate, and subject to heating and reflux reaction for 45 minutes to obtain 7.5g drospirenone of Formula VII.

### Example 5

(1) 5g of 3β,5-dihydroxy-6β,7β,15β,16β-dimethylene-5β-androstane-17,20-epoxy (compound of Formula I) was dissolved in 50ml pyridine, added into 50ml pyridine solution containing 5g chromic anhydride, evenly stirred, subject to reaction at 80°C for 1 hour, placed overnight at room temperature, put into ice water, extracted and filtered to obtain 4.6g oxide of Formula II.
(2) 5g of the oxide of Formula II (5g oxide of Formula II prepared by step (1)), 50ml of dichloromethane, 5ml of triethyl orthoformate were evenly mixed with 10ml glycol, added with 0.5g sodium bisulfate, and stirred at room temperature for 3 hours to obtain 5.5g ketal product of Formula III.
(3) The solution of 500ml methanol and 20g sodium methoxide was dripped with 70ml methanol solution containing 60ml dimethyl malonate at 0 °C, subject to heating and reflux reaction for 30 minutes, cooled down to room temperature, added with 20 g ketal product of Formula III (20g ketal product of Formula III prepared by step (2)), stirred at room temperature for 3 hours, and then subject to heating and reflux reaction for 20 hours to obtain the compound of Formula IV.
(4) All of compound of Formula IV produced in last step was mixed with 120ml DMF, 20ml H₂O and 24g lithium bromide, and subject to reflux reaction for 3.5 hours to obtain 20.5g lactone compound of Formula V.
(5) The lactone compound of Formula V of 20g was dissolved in 100 ml pyridine, added with 10g methane sulfonyl chloride, and subject to reaction at 0°C -5°C for 1 hour to obtain the sulfonyl compound of Formula VI.
(6) All of sulfonyl compound of Formula VI produced in the last step was mixed with 150ml glacial acetic acid and 25g sodium acetate, and subject to heating and reflux reaction for 30 minutes to obtain 17g drospirenone of Formula VII.

### Example 6

(1) 5g of 3β,5-dihydroxy-6β,7β,15β,16β-dimethylene-5β-androstane-17,20-epoxy (compound of Formula I) was dissolved in 50ml pyridine, added into 50ml pyridine solution containing 5g chromic anhydride, evenly stirred, subject to reaction at 10 °C for 2.5 hours, placed overnight at room temperature, put into ice water, extracted and filtered to obtain 4.5g oxide of Formula II.
(2) 5g of the oxide of Formula II (5g oxide of Formula II prepared by step (1)), 50ml of dichloromethane, 5ml of triethyl orthoformate are evenly mixed with 10ml glycol, added with 0.5g sodium bisulfate and stirred at 0°C for 15 hours to obtain 5.4g ketal product of Formula III.
(3) The solution of 500ml methanol and 20g sodium methoxide was dripped with 70ml methanol solution containing 60ml dimethyl malonate at -10 °C, subject to heating and reflux reaction for 30 minutes, cooled down to room temperature, added with 20 g ketal product of Formula III (20g ketal product of Formula III prepared by step (2)), stirred at 0 °C for 3 hours, and then subject to heating and reflux reaction for 15 hours to obtain the compound of Formula IV.
(4) All of compound of Formula IV produced in last step was mixed with 120ml DMF, 20ml H₂O and 24g lithium bromide, and subject to reaction at 100°C for 4 hours to obtain 20.2g lactone compound of Formula V.
(5) 20g of the lactone compound of Formula V was dissolved in 100 ml pyridine, added with 10g methane sulfonyl chloride, and subject to reaction at 50°C for 3 hours to obtain the sulfonyl compound of Formula VI.
(6) All of sulfonyl compound of Formula VI produced in the last step is mixed with 150ml glacial acetic acid and 25g sodium acetate, and subject to heating and reflux reaction for 30 minutes to obtain 16.5g drospirenone of Formula VII.

### Example 7

(1) 5g of 3β,5-dihydroxy-6β,7β,15β,16β-dimethylene-5β-androstane-17,20-epoxy (compound of Formula I) was dissolved in 50ml pyridine, added into 50ml pyridine solution containing 5g chromic anhydride, evenly stirred, subject to reaction at 65 °C for 1.5 hours, placed overnight at room temperature, put into ice water, extracted and filtered to obtain 4.4g oxide of Formula II.
(2) 5g of the oxide of Formula II (5g oxide in Formula II prepared by step (1)), 50ml of dichloromethane, 5ml of triethyl orthoformate were evenly mixed with 10ml glycol, added with 0.5g sodium bisulfate and stirred at 55 °C for 10 hours to obtain 5.45g ketal product of Formula III.
(3) The solution of 500ml methanol and 20g sodium methoxide was dripped with 70ml methanol solution containing 60ml dimethyl malonate at 15°C, subject to heating and reflux reaction for 30 min, cooled down to room temperature, added with 20g ketal product of Formula III (20g ketal product of Formula III prepared by step (2)), stirred at 55°C for 3 hours, and then subject to heating and reflux reaction for 10h to obtain the compound of Formula IV.
(4) All of compound of Formula IV produced in last step was mixed with 120ml DMF, 20ml H₂O and 24g lithium bromide, and subject to reaction at 140°C for 2 hours to obtain 20.3g lactone compound of Formula V.
(5) The lactone compound of Formula V of 20g was dissolved in 100 ml pyridine, added with 10g methane sulfonyl chloride, and subject to reaction at 65 °C for 2.5 hours to obtain the sulfonyl compound of Formula VI.
(6) All of sulfonyl compound of Formula VI produced in the last step was mixed with 150ml glacial acetic acid and 25g sodium acetate, and subject to heating and reflux reaction for 50 min to obtain 16.6g drospirenone of Formula VII.

### Example 8

(1) 5g of 3β,5-dihydroxy-6β,7β,15β,16β-dimethylene-5β-androstane-17,20-epoxy (compound of Formula I) was dissolved in 50ml pyridine, added into 50ml pyridine solution containing 5g chromic anhydride, evenly stirred, subject to reaction at 35 °C for 2 hours, placed overnight at room temperature, put into ice water, extracted and filtered to obtain 4.35g oxide of Formula II.
(2) 5g of the oxide of Formula II (5g oxide of Formula II prepared by step (1)), 50ml of dichloromethane, 5ml of triethyl orthoformate were mixed with 48ml glycol, added with 0.5g sodium bisulfate and stirred at 80°C for 1 hour to obtain 5.5g ketal product of Formula III.
(3) The solution of 500ml methanol and 20g sodium methoxide was dripped with 70ml methanol solution containing 60ml dimethyl malonate at 30 °C, subject to heating and reflux reaction for 30 minutes, cooled down to room temperature, added with 20 g ketal product of Formula III (20g ketal product of Formula III prepared by step (2)), stirred at 80°C for 3 hours, and then subject to heating and reflux reaction for 8 hours to obtain the compound of Formula IV.
(4) All of compound of Formula IV produced in last step was mixed with 120ml DMF, 20ml H₂O and 24g lithium bromide, and subject to reaction at 180°C for 0.5 hour to obtain 20.1g lactone compound of Formula V.
(5) The lactone compound of Formula V of 20g was dissolved in 100 ml pyridine, added with 10g methane sulfonyl chloride, and subject to reaction at 80 °C for 1.5 hour to obtain the sulfonyl compound of Formula VI.
(6) All of sulfonyl compound of Formula VI produced in the last step was mixed with 150ml glacial acetic acid and 25g sodium acetate, and subject to heating and reflux reaction for 60 minutes to obtain 16.8g drospirenone of Formula VII.

## Claims

1. A preparation method of drospirenone, comprising the following steps:
Providing the compound 3β,5-dihydroxy-6β,7β,15β,16β-dimethylene-5β-androstane-17,20-epoxy of Formula I as starting material; oxidizing the hydroxyl group at the 3-position to obtain an oxide of Formula II, and then protecting the 3-ketone group by ketalization to obtain the ketal product of Formula III; the obtained ketal product being converted into the compound of Formula IV by a condensation reaction, and then obtaining the lactone compound of Formula V by decarboxylation; the hydroxyl at the 5-position being subject to sulfonation to obtain the sulfonyl compound of Formula VI; and the deketalization and desulphonation being performed in the reaction system of glacial acetic acid and sodium acetate to produce the 3-keto-4-ene compound, being drospirenone of Formula VII 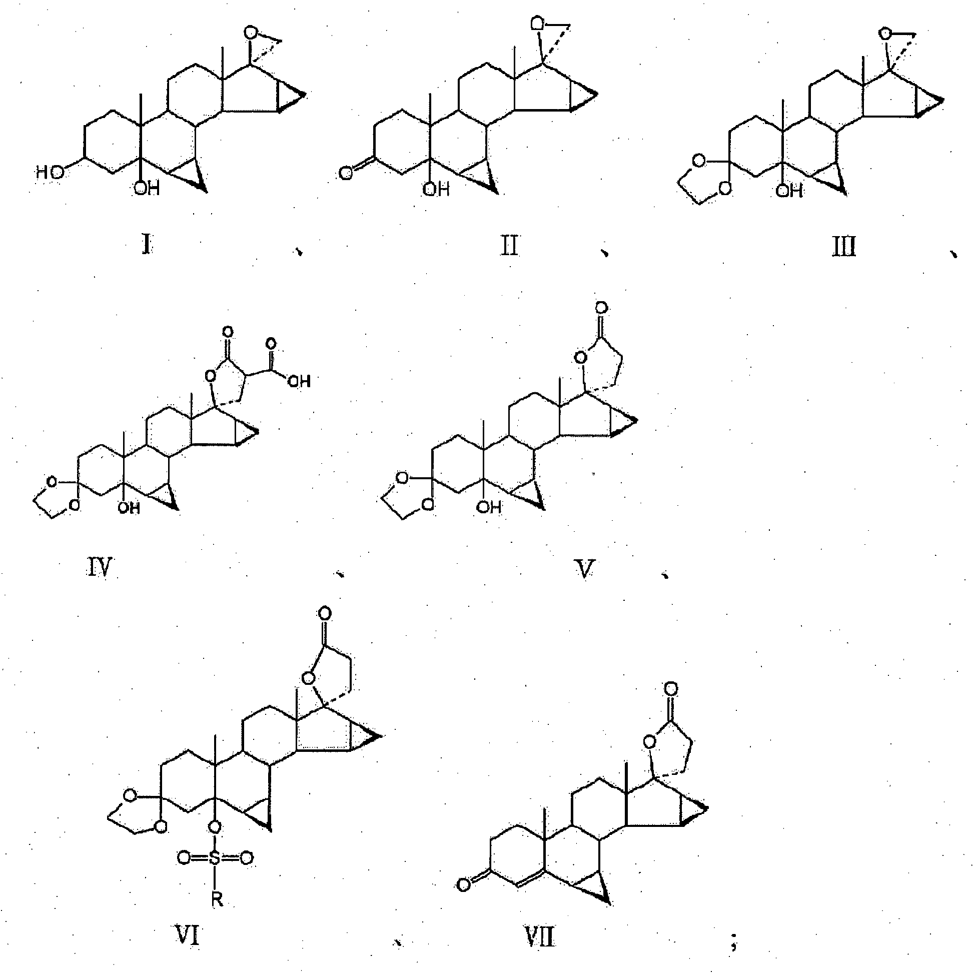 in formula VI, R being C₁-C₁₀ alkyl, **characterized in that** the method comprises the following steps:
(1) 3β,5-dihydroxy-6β,7β,15β,16β-dimethylene-5β-androstane-17,20-epoxy of Formula I is taken as stating material, and reacts with an oxidant in solvent at 0°C -80°C to obtain the oxide of Formula II;
(2) the oxide of Formula II, glycol and acid catalyst are mixed and subject to reflux reaction or reaction with dehydration agent at 0°C -80°C to obtain the ketal product of Formula III;
(3) an alcoholic solution of dimethyl malonate is blended with miscible liquids of alcohol and sodium alcoholate at -10°C to 30 °C, and then subject to heating and reflux reaction, the reactant being cooled down, put into the ketal product in Formula III, and subject to reaction at 0 °C -80°C to produce the compound of Formula IV;
(4) the compound of Formula IV reacts with alkali metal halide salt in the presence of N,N -dimethylformamide and is subject to reflux reaction or reaction at 100°C -180°C to obtain the lactone compound of Formula V;
(5) the lactone compound of Formula V reacts with sulfonyl chloride compound in the presence of pyridine at 0°C -80°C to obtain the sulfonyl compound of the Formula VI;
(6) the sulfonyl compound of Formula VI is subject to reflux reaction in the reaction system of glacial acetic acid and sodium acetate to produce drospirenone of Formula VII.

2. The preparation method according to Claim 1, wherein the oxidant of step (1) is selected from one of sulfuric acid solution containing chromic anhydride, pyridine solution containing chromic anhydride, or N-bromosuccinimide.

3. The preparation method according to Claim 1, wherein the solvent in step (1) is selected from one or more of acetone, pyridine, methanol and tert-butyl alcohol.

4. The preparation method according to Claim 1, wherein the acid catalyst in step (2) is selected from one of p-toluenesulfonic acid, boron trifluoride-diethyl ether solution, sulfuric acid, sodium bisulfate; or the said dehydrant is triethyl orthoformate.

5. The preparation method according to Claim 1 or Claim 4, wherein the weight ratio of oxide of Formula II to acid catalyst said in the step (2) is 1:0.00005-0.1; and the weight ratio of oxide of Formula II to glycol is 1:2-10.

6. The preparation method according to Claim 1, wherein the alcohol in step (3) is methanol or ethanol; and the sodium alcoholate is sodium methoxide or sodium ethoxide.

7. The preparation method according to Claim 1, wherein the alkali metal halide salt in step (4) is selected from lithium bromide or sodium chloride.

8. The preparation method according to Claim 1, wherein the sulfonyl chloride compound in step (5) is selected from one of benzene sulfonyl chloride, p-toluenesulfonyl chloride and methanesulfonyl chloride; and R in Formula VI is selected from one of phenyl, tolyl and methyl.

9. The preparation method according to Claim 1, wherein the sodium acetate accounts for 9%-20% mass percent in the reaction system in step (6).

## Patentansprüche

1. Verfahren zur Herstellung von Drospirenon, das folgende Schritte umfasst:
Bereitstellen der Verbindung 3β,5-Dihydroxy-6β,7β,15β,16β-dimethylen-5β-androstan-17,20-epoxid der Formel I als Ausgangsmaterial; Oxidieren der Hydroxygruppe an der Position 3, um ein Oxid der Formel II zu erhalten, und anschließend Schützen der 3-Ketongruppe durch Ketalisierung, um das Ketalprodukt der Formel III zu erhalten; wobei das erhaltene Ketalprodukt durch eine Kondensationsreaktion in die Verbindung der Formel IV umgewandelt wird, und anschließend Erhalt der Lactonverbindung der Formel V durch Decarboxylierung; wobei das Hydroxy an der Position 5 sulfoniert wird, um die Sulfonylverbindung der Formel VI zu erhalten; und wobei die Deketalisierung und Desulfonierung im Reaktionssystem aus Eisessig und Natriumacetat erfolgen, um die 3-Keto-4-en-Verbindung, bei der es sich um Drospirenon der Formel VII handelt, zu erhalten 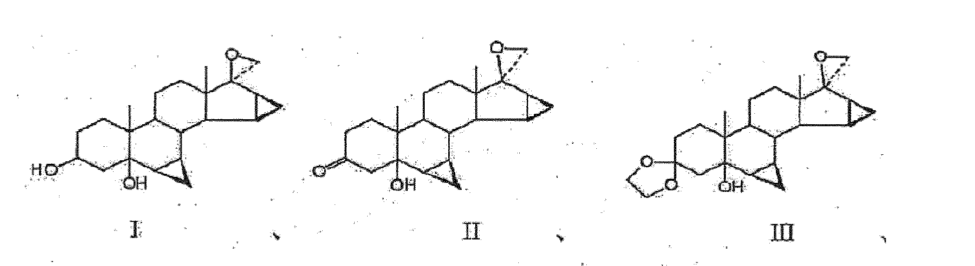 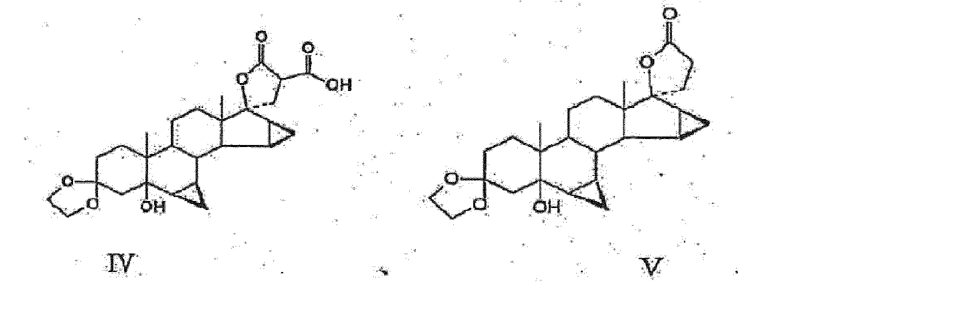 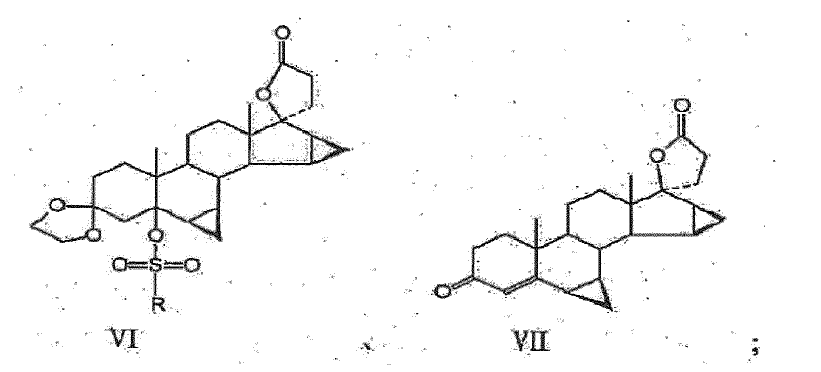 wobei R in Formel VI C₁-C₁₀-Alkyl ist, **dadurch gekennzeichnet, dass** das Verfahren folgende Schritte umfasst:
(1) 3β,5-Dihydroxy-6β,7β,15β,16β-dimethylen-5β-androstan-17,20-epoxid der Formel I wird als Ausgangsmaterial genommen und reagiert in Lösungsmittel bei 0°C-80°C mit einem Oxidationsmittel, um das Oxid der Formel II zu erhalten;
(2) das Oxid der Formel II, Glykol und saurer Katalysator werden gemischt und unter Rückfluss oder mit Dehydratisierungsmittel bei 0°C-80°C zur Reaktion gebracht, um das Ketalprodukt der Formel III zu erhalten;
(3) eine alkoholische Lösung von Dimethylmalonat wird bei -10°C bis 30°C mit mischbaren Flüssigkeiten aus Alkohol und Natriumalkoholat gemischt und anschließend erhitzt und unter Rückfluss zur Reaktion gebracht, wobei der Reaktant abgekühlt wird, in das Ketalprodukt in Formel III eingebracht wird und bei 0°C-80°C zur Reaktion gebracht wird, um die Verbindung der Formel IV zu erzeugen;
(4) die Verbindung der Formel IV reagiert mit Alkalimetallhalogenid-Salz in Gegenwart von N,N-Dimethylformamid und wird unter Rückfluss oder bei 100°C-180°C zur Reaktion gebracht, um die Lactonverbindung der Formel V zu erhalten;
(5) die Lactonverbindung der Formel V reagiert mit Sulfonylchloridverbindung in Gegenwart von Pyridin bei 0°C-80°C, um die Sulfonylverbindung der Formel VI zu erhalten;
(6) die Sulfonylverbindung der Formel VI wird im Reaktionssystem aus Eisessig und Natriumacetat unter Rückfluss zur Reaktion gebracht, um Drospirenon der Formel VII zu erzeugen.

2. Herstellungsverfahren nach Anspruch 1, wobei das Oxidationsmittel von Schritt (1) ausgewählt ist aus Chrom(VI)-oxid enthaltender Schwefelsäurelösung, Chrom(VI)-oxid enthaltender Pyridinlösung oder N-Bromsuccinimid.

3. Herstellungsverfahren nach Anspruch 1, wobei das Lösungsmittel in Schritt (1) ausgewählt ist aus Aceton, Pyridin, Methanol und/oder tert-Butylalkohol.

4. Herstellungsverfahren nach Anspruch 1, wobei der saure Katalysator in Schritt (2) ausgewählt ist aus p-Toluensulfonsäure, Bortrifluoriddiethylether-Lösung, Schwefelsäure oder Natriumbisulfat; oder das Dehydratationsmittel Triethylorthoformat ist.

5. Herstellungsverfahren nach Anspruch 1 oder Anspruch 4, wobei das Gewichtsverhältnis von Oxid der Formel II zu in Schritt (2) genanntem saurem Katalysator 1 : 0,00005-0,1 beträgt; und das Gewichtsverhältnis von Oxid der Formel II zu Glykol 1 : 2-10 beträgt.

6. Herstellungsverfahren nach Anspruch 1, wobei der Alkohol in Schritt (3) Methanol oder Ethanol ist; und das Natriumalkoholat Natriummethoxid oder Natriumethoxid ist.

7. Herstellungsverfahren nach Anspruch 1, wobei das Alkalimetallhalogenid-Salz in Schritt (4) aus Lithiumbromid oder Natriumchlorid ausgewählt ist.

8. Herstellungsverfahren nach Anspruch 1, wobei die Sulfonylchloridverbindung in Schritt (5) ausgewählt ist aus Benzensulfonylchlorid, p-Toluensulfonylchlorid oder Methansulfonylchlorid; und R in der Formel VI ausgewählt ist aus Phenyl, Tolyl oder Methyl.

9. Herstellungsverfahren nach Anspruch 1, wobei das Natriumacetat 9-20 Masseprozent des Reaktionssystems in Schritt (6) ausmacht.

## Revendications

1. Fournir le composé 3β,5-dihydroxy-6β,7β,15β,16β-diméthylène-5β-androstane-17,20-époxy de Formule I comme substance de départ ; oxider le groupe hydroxyle à la position 3 afin d'obtenir l'oxyde de Formule II, puis protéger le groupe 3-cétone par cétalisation afin d'obtenir le produit cétal de Formule III ; le produit cétal obtenu étant transformé en le composé de Formule IV par réaction de condensation, puis obtenant le composé de lactone de Formule V par décarboxylation ; l'hydroxyle en position 5 étant sujet à sulfonation et estérification afin d'obtenir le composé de sulfonyle de Formule VI ; et la décétalisation et la désulfonation étant effectuées dans le système réactionnel d'acide acétique glacial et d'acétate de sodium afin de produire un composé 3-céto-4-ène, étant la drospirénone de Formule VII 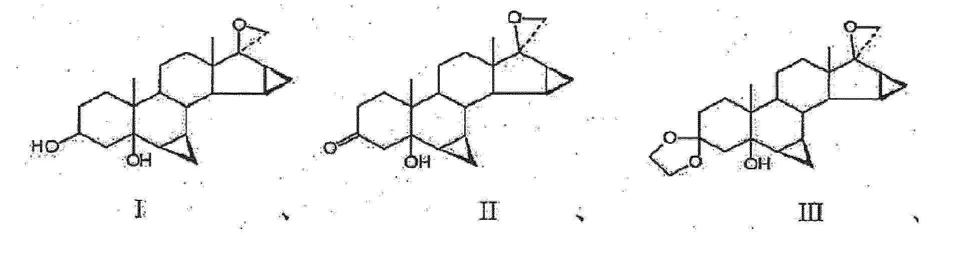 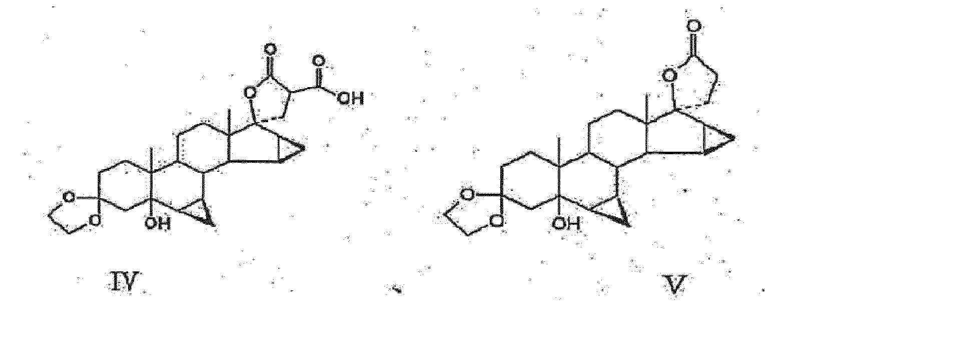 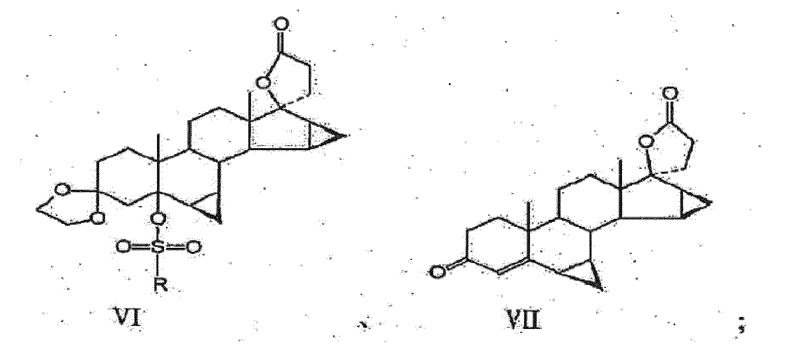 dans la formule VI, R étant un alkyle C₁-C₁₀, **caractérisé en ce que** le procédé comprend les étapes suivantes :
(1) le 3β,5-dihydroxy-6β,7β,15β,16β-diméthylène-5β-androstane-17,20 -époxy de formule 1 est pris comme substance de départ et réagit avec un oxydant en solvant à 0°C -80 °C afin d'obtenir l'oxyde de formule II ;
(2) l'oxyde de formule II, le glycol et le catalysateur acide sont mélangés et soumis au reflux ou à une réaction avec un agent de déshydratation à 0°C -80°C pour obtenir le composé cétal de formule III ;
(3) une solution alcoolique de malonate de diméthyle est mélangée avec des liquides miscibles d'alcool et d'alcoolate de sodium de -10°C à 30 °C, puis soumis au chauffage et au reflux, le réactif étant refroidi, mis dans le composé cétal de formule III et soumis à réaction à 0°C -80°C afin de produire le composé de formule IV ;
(4) le composé de formule IV réagit avec un sel d'halogénure de métal alcalin en présence de N,N -diméthylformamide et est soumis au reflux ou à une réaction à 100°C -180°C afin d'obtenir le composé de lactone de formule V ;
(5) le composé de lactone de formule V réagit avec un composé de chlorure de sulfonyle en présence de pyridine à 0°C -80°C afin d'obtenir le composé de sulfonyle de formule VI ;
(6) le composé de sulfonyle de formule VI est soumis au reflux dans le système réactionnel d'acide acétique glacial et d'acétate de sodium afin de produire la drospirénone de Formule VII.

2. Procédé de préparation selon la revendication 1, ledit oxydant de l'étape (1) étant sélectionné parmi : une solution d'acide sulfurique contenant de l'anhydride chromique, une solution de pyridine contenant de l'anhydride chromique ou de la N-bromosuccinimide.

3. Procédé de préparation selon la revendication 1, ledit solvant de l'étape (1) étant sélectionné entre un ou plusieurs parmi : l'acétone, la pyridine, le méthanol et l'acool tert-butylique.

4. Procédé de préparation selon la revendication 1, ledit catalysateur acide de l'étape (2) étant sélectionné parmi : l'acide paratoluènesulfonique, une solution d'éther diéthylique et de trifluorure de bore, l'acide sulfurique, le bisulfate de sodium ; ou ledit déshydratant est l'orthoformiate de triéthyle.

5. Procédé de préparation selon la revendication 1 ou la revendication 4, le rapport de poids de l'oxyde de formule II avec ledit catalysateur acide de l'étape (2) est de 1:0,00005-0,1 ; et le rapport de poids de l'oxyde de formule II avec le glycol est 1:2-10.

6. Procédé de préparation selon la revendication 1, ledit alcool de l'étape (3) étant du méthanol ou de l'éthanol ; et l'alcoolate de sodium étant du méthanolate de sodium ou de l'éthanolate de sodium.

7. Procédé de préparation selon la revendication 1, ledit sel d'halogénure de métal alcalin de l'étape (4) étant sélectionné parmi : du bromure de lithium ou du chlorure de sodium.

8. Procédé de préparation selon la revendication 1, ledit composé de chlorure de sulfonyle de l'étape (5) étant sélectionné parmi : le chlorure de sulfonyle de benzène, le chlorure de paratoluènesulfonyle et le chlorure de méthanesulfonyle ; et R dans la formule VI étant sélectionné parmi les groupes suivants : phényle, tolyle et méthyle.

9. Procédé de préparation selon la revendication 1, l'acétate de sodium représentant 9%-20% en masse dans le système réactionnel de l'étape (6).
